Europäisches Patentamt

**European Patent Office**

**Office européen des brevets**

(11) Veröffentlichungsnummer: **0 040 592**
**B1**

(12) **EUROPÄISCHE PATENTSCHRIFT**

(45) Veröffentlichungstag der Patentschrift:
**20.03.85**

(51) Int. Cl.⁴: **A 61 M 5/14**

(21) Anmeldenummer: **81890077.1**

(22) Anmeldetag: **11.05.81**

(54) **Automatische Infusionspumpe.**

(30) Priorität: **16.05.80 DE 3018641**

(43) Veröffentlichungstag der Anmeldung:
**25.11.81 Patentblatt 81/47**

(45) Bekanntmachung des Hinweises auf die Patenterteilung:
**20.03.85 Patentblatt 85/12**

(84) Benannte Vertragsstaaten:
**AT BE CH FR GB IT LI NL SE**

(56) Entgegenhaltungen:
**DE - A - 2 318 655**
**DE - A - 2 529 636**
**DE - A - 2 733 694**
**DE - A - 2 852 041**
**FR - A - 2 350 107**
**US - A - 2 979 055**
**US - A - 3 456 648**
**US - A - 3 990 444**
**US - A - 4 105 028**
**US - A - 4 191 184**

**Münch. med. Wschr. 121 (1979) Nr. 24, S. 821-824**

(73) Patentinhaber: **Rodler, Hans, Pehamweg 3-5,
A-8053 Graz-Neuhart (AT)**

(72) Erfinder: **Rodler, Hans, Pehamweg 3-5,
A-8053 Graz-Neuhart (AT)**

**Beschreibung**

Infusionspumpe zur intravenösen oder intraarteriellen Infusion von flüssigen Medikamenten mit einer elektrisch betriebenen Pumpe und einer elektronischen Mengensteuerung.

Elektrisch betriebene Infusionspumpen mit elektronischer Mengensteuerung sind bekannt. Bei diesen wird vorzugsweise die erforderliche Menge mittels einer Skale vorgewählt und über eine Schlauchpumpe dem Patienten zugeführt (DE-A-2 318 655). Eine andere Form von elektrisch betriebenen Infusionspumpen arbeitet mit einer Kolbenpumpe, wobei die Geschwindigkeit des Kolbens elektronisch gesteuert wird (DE-A-2 529 636).

Durch die Pharmazie stehen Medikamente mit spontanem Wirkungsmechanismus zur Verfügung. Diese haben kurzfristige Wirkungen und schon eine geringe Überdosierung kann unter Umständen den Tod herbeiführen. Ausserdem gibt es blutdrucksenkende Mittel, die besonders in der Chirurgie angewendet werden, um Blutungen während der Operation zu vermeiden. Um einen bestimmten Blutdruckwert einzustellen, muss dieses Präparat dem Patienten ununterbrochen in einer bestimmten Menge zugeführt werden. Da eine zu weitgehende Senkung eine kritische Situation für den Patienten darstellt, muss daher die Infusion ständig von einem Arzt kontrolliert werden. Ein anderes Anwendungsgebiet dieser Mittel ist bei Schlaganfällen oder nach Operationen bei kritischen Blutdrucksituationen auf Intensivstationen. Um das Präparat wirkungsvoll einzusetzen, muss daher auch hier ständig die Menge und der Blutdruck von einem Arzt kontrolliert werden. Auch temperatursenkende Mittel machen eine ständige Kontrolle der Infusionsmittel notwendig. Bei den bisher bekannten Anordnungen konnte nur eine Menge fix vorgewählt werden und durch ständige Kontrolle überwacht werden, ob die Menge den Erfordernissen entspricht. Aus der Münchner med. Wochenschrift 121 (1979) Nr. 24, Seiten 821–824 ist bekannt, zur Therapie von schweren Verbrennungen proportional oder dreipunktgeregelte Infusionspumpen, die vom Überwachungscomputer gesteuert werden, einzusetzen. Es wird hierbei in Abhängigkeit vom Venendruck bei zu grosser Drucksenkung die Infusionsgeschwindigkeit halbiert, respektive im umgekehrten Falle wieder erhöht. Da in diesem Falle keine spontan wirkenden Medikamente angewendet werden, genügt diese einfache Form der Regelung, um die angestrebte Schockkompensation zu erreichen.

Der Erfindung liegt die Aufgabe zugrunde, eine automatische Infusionseinrichtung zu schaffen, die es erlaubt, auch Medikamente mit spontanem Wirkungsmechanismus ohne ständige ärztliche Kontrolle einzusetzen, welche auch im Operationsbereich bei narkotisierten und bewusstlosen Patienten sicher eingesetzt werden kann und die auch bei Fehlbedienung für den Patienten keine Gefährdung darstellt und bei der eine falsche Dosierung vom Bedienungspersonal nicht möglich ist. Auch ist es Aufgabe der Erfindung, die erforderliche Infusionsmenge für eine bestimmte vorwählbare physikalische Messgrösse zu finden und diese Menge zur Aufrechterhaltung dieser vorgewählten physikalischen Messgrösse dem Patienten zuzuführen.

Die Aufgabe wird entsprechend den Merkmalen des kennzeichnenden Teils des Patentanspruches 1 gelöst. Somit wird der elektrischen Infusionspumpe, die vorwiegend mit einem Schrittschaltmotor oder Synchronmotor betrieben wird, eine Frequenz zugeführt, die durch eine digitale elektronische Steuereinrichtung generiert wird, wobei die vom Patienten abgenommene physikalische Messgrösse diese Frequenz beeinflusst. Hiezu wird die physikalische Messgrösse mit einem einstellbaren Sollwert verglichen. Ausserdem wird der Vergleich innerhalb einer oberen und unteren Grösse negiert. Ausserdem wird die Motorfrequenz nicht sofort nach Feststellung einer Abweichung sondern erst nach einer einstellbaren Zeitverzögerung zu einem höheren Wert geführt und damit zu einer grösseren Infusionsmenge, so dass der menschliche Körper Zeit hat, auf das Präparat zu reagieren. Gleichzeitig wird dadurch erreicht, dass kurzzeitige physikalische Änderungen, die durch Bewegungen, Artefakte und dergleichen hervorgerufen werden können, nicht berücksichtigt werden. Weiters erfolgt der Anstieg der Infusionsmenge und der Motorfrequenz nicht spontan sondern in Stufen, die durch die Zeitverzögerung ein langsames Einschleichen zur erforderlichen Menge ermöglichen. Die Infusionsmenge steigt also stufenförmig bis zu dem Punkt an, in dem der Körper die entsprechende Reaktion zeigt und die physikalische Grösse mit dem eingestellten Sollwert übereinstimmt. Eine Sicherheitsmassnahme, um Spontanreaktionen des Patienten zu berücksichtigen, besteht darin, dass bei Überschreiten der physikalischen Grösse in Richtung des Wirkungsmechanismus die Infusionsmenge auf den halben Wert gesenkt wird und bei weiterem Überschreiten einer dritten Grenze in Richtung des Wirkungsmechanismus die Infusionspumpe gestoppt und Alarm gegeben wird. Hiedurch ist sichergestellt, dass bei unkontrollierten Reaktionen des Patienten, die bei gewissen Medikamenten möglich sind, die Infusionsmenge entweder verkleinert oder sofort abgesetzt wird. Gleichzeitig wird die tatsächlich durchfliessende Infusionsmenge gemessen und bei Fehlmengen ebenfalls die Pumpe stillgesetzt und Alarm gegeben. Hiedurch wird vermieden, dass ein verstopftes Infusionsbesteck Fehlinterpretationen der Pumpe hervorrufen. Gleichzeitig wird der Druck im Infusionsschlauch ebenfalls zu demselben Zweck für die Steuerung und Sicherheitsabschaltung herangezogen. Bei Abfall des Druckes unter einen gewissen Wert, wird ebenfalls Alarm gegeben, da dies bedeutet, dass das Infusionsbesteck undicht geworden ist und das Infusionspräparat nicht dem Patienten zugute kommt. Eine weitere Sicherheitsmassnahme ist das Überwachen von Luftblasen mit einer Lichtschranke. Der Frequenzgenerator ist ein gesteuerter digitaler Oszillator, der von einer digitalen Schaltung mit entsprechenden Steuerwerten versorgt wird, damit ein kontinuierlicher oder wenn gewünscht ein exponentialer Anstieg der Infusionsmenge und der Frequenz des Oszillators erfolgt.

Die vom Patienten abgenommene physikalische Messgrösse wird durch Differenzbildung mit dem Sollwert verglichen und solange eine Differenz besteht, bewirkt der am Ausgang vorhandene Messwert, dass die Oszillatorfrequenz der Motorfrequenzsteuerung unter Berücksichtigung einer Zeitverzögerung ansteigt, wobei die Grösse der Stufen konstruktiv vorgegeben ist. Aus der Durchflussmenge wird ebenfalls ein Messwert gewonnen, der mit der Motorfrequenz verglichen wird und im abweichenden Fall ebenfalls zum Abschalten des Oszillators führt. Nimmt man an, dass als Infusionsmittel ein blutdrucksenkendes Mittel dient, dann wird vom Patienten der arterielle Blutdruck abgenommen, den ein Messwertumformer in ein elektrisches Signal verwandelt und mit dem Sollwert einen Differenzwert bildet. Dieser Wert bewirkt nun, dass die Oszillatorfrequenz eingeschaltet wird und in der niedersten Stufe zu schwingen beginnt. Besteht nach Ablauf einer vorgegebenen, von aussen einstellbaren Verzögerungszeit der Differenzwert immer noch, dann wird die nächste Stufe geschaltet usw. bis zu dem Punkt, an dem der Differenzwert zwischen Soll- und Istwert gleich Null ist. Da die Infusionsmenge langsam ansteigt, wird dieser Nullwert längere Zeit aufrecht bleiben. Tritt nun nach einer gewissen Zeit ein Abfall des Blutdruckes unter eine Toleranzgrenze von z.B. 20% ein, tritt also in diesem Falle eine negative Differenz zwischen Soll- und Istwert auf, so bewirkt dies, dass die Oszillatorfrequenz auf den halben Wert zurückgeht. Sinkt der Blutdruckwert weiter über eine ebenfalls vorgegebene Drittgrenze, ist also die negative Differenz zwischen Soll- und Istwert grösser, so schaltet der Oszillator automatisch ab und die Infusionsmenge wird Null. Gleichzeitig wird hiebei eine Alarmeinrichtung in Tätigkeit gesetzt. Sinkt der Wert nur unter eine erste Grenze, so wird in der Zeitverzögerung nur um eine Stufe zurückgeschaltet. Es ist also in diesem Falle im aufsteigenden Bereich eine Grenze, die eine Hysteresis darstellt, vorgesehen, während im abfallenden Bereich drei Grenzen vorgesehen sind, wobei die erste die Hysteresisgrenze darstellt, innerhalb welcher mit entsprechenden Zeitverzögerungen die nächste untere Stufe gewählt wird, während bei Überschreiten der zweiten Stufe auf den halben Wert geschaltet wird und nach Überschreiten der dritten Grenze Stop und Alarm gegeben wird. Durch die Zeitverzögerung und dem Amplitudenhysteresewert wird ausserdem vermieden, dass Regelschwingungen eintreten. Die ersten beiden Grenzen der Hysterese können entweder im digitalen Bereich oder im Eingangsverstärker selbst gebildet werden. Ebenso können die zweite und die dritte Grenze nach unten durch entsprechende Eingangsdifferenzverstärker, aber auch in der weiteren digitalen Steuerung realisiert werden. Der Vorteil dieser Anordnung besteht insbesonders darin, dass in Intensivstationen, in denen eine dauernde Überwachung des Blutdruckes erforderlich ist, der Patient nicht nur überwacht sondern auch gleichzeitig das entsprechende lebensrettende Präparat zugeführt erhält. Gerade bei kritischen Blutdruckschwankungen nach Operationen oder nach Schlaganfällen können kritische Werte nicht nur zu Gehirnschäden und damit Lähmungen u. dgl. sondern auch zum Erblinden führen. Während der Operation kann durch eine gesicherte Blutdrucksenkung übermässiger Blutverlust vermieden werden und damit bessere Heilaussichten gesichert werden.

Weitere erfindungswesentliche Merkmale sind in der Figurenbeschreibung näher erläutert.

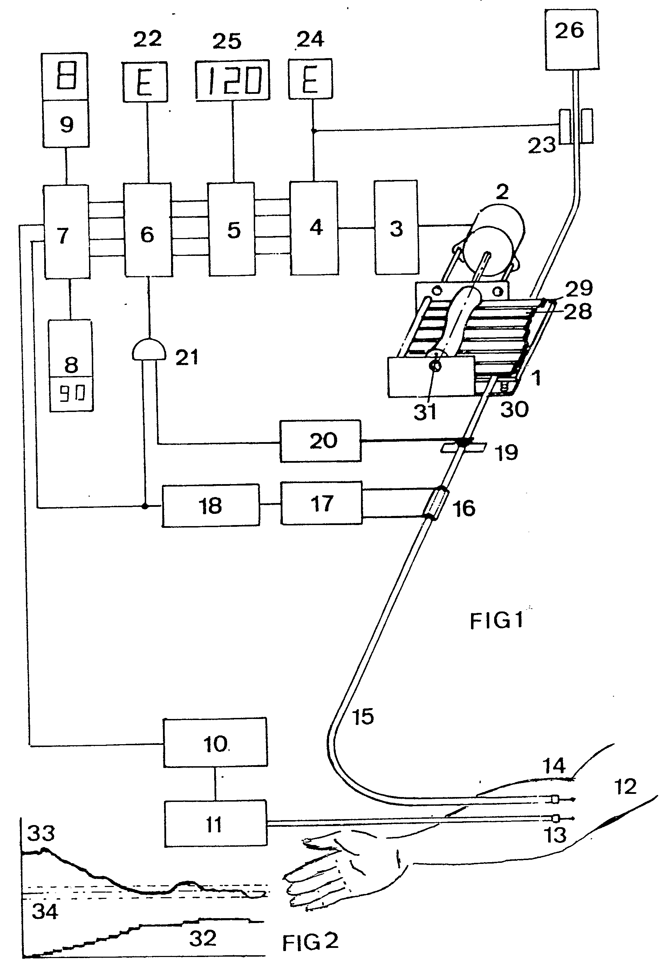

Fig. 1 zeigt ein Schaltbild der Infusionspumpe, Fig. 2 zeigt die Regelkurve.

In Fig. 1 ist 1 die Peristaltikpumpe, die mittels den Lamellen 28 und der Gegenhalteplatte 29 den Infusionsschlauch 15 durch Anpressen einer Lamelle nach der anderen durch die schraubenförmige Spindel 31 die Pumpwirkung hervorruft. 2 ist ein Schrittschaltmotor, der die Peristaltikpumpe antreibt. Dieser Schrittschaltmotor wird durch die Elektronik 3, die am Eingang einen einphasigen Wechselstrom und am Ausgang einen mehrphasigen Wechselstrom erzeugt, angetrieben. Die im Behälter 26 befindliche Infusionsflüssigkeit gelangt nun durch den Schlauch 15 über eine Druckmesseinrichtung 19 und über eine Mengenmesseinrichtung 16 zum Patienten 12 und wird mittels einer Kanüle 14 in die Vene des Patienten eingeführt. Über die Kanüle 13 wird vom Patienten 12 gleichzeitig die Blutdruckgrösse aus der Arterie abgenommen und mittels der Druckwandeleinrichtung 11 in ein elektrisches Signal und durch den Analog-Digitalwandler 10 in ein digitales Signal umgewandelt. Die Mengenmesseinrichtung 16 wird durch das Mengenwandelgerät 17 in ein elektrisches Signal und durch den Analog-Digitalwandler 18 in ein Digitalsignal verwandelt. Die Druckmesseinrichtung 19 steuert einen Schalter 20, der bei zu niedrigem Druck anspricht. Die Mengengrösse und die Druckgrösse sind durch ein Undgatter 21 mit der Steuereinheit 6 verbunden. Diese Steuereinheit stoppt den Pumpenmotor 2 und gibt durch die Anzeige 22 Alarm, wenn der Druck im Sensor 19 Null und die Durchflussmenge Null ist oder wenn die Durchflussmenge Null und der Druck hoch ist oder wenn der Druck Null und die Durchflussmenge hoch ist. Diese Funktion ist durch das Undgatter 21 gegeben. Die Logik 6 ist also nur dann nicht blockiert, wenn der Druck 19 eine gewisse Grösse aufweist und die Durchflussmenge 16 die richtige Grösse aufweist. Der digitale Blutdruckwert des Analog-Digitalwandlers 10 und der digitale Mengenwert des Analog-Digitalwandlers 18 wird der Logik 7, die z.B. in dem Mikroprozessor 8085, 8048, Z 80, 6000 oder ähnlichem enthalten ist, zugeführt. 8 ist der einstellbare Druck-Sollwertgeber, der die Vergleichsgrösse für den gemessenen Druck für die Logik 7 bildet und anzeigt. In der Logik 7 sind die unteren und oberen Grenzwerte der prozentuellen Abweichung vom Sollwert zum Istwert festgelegt, ebenso ist in 7 die Hysteresegrösse programmiert. 9 bildet die von aussen einstellbare Zeitverzögerung, nach welcher an den Steuerbaustein 6, der durch den Mikroprozessor 8085, 8048, Z 80, 6000 oder ähnlichem gebildet wird, das Signal weitergegeben wird. Die Logik 7 kann zweckmässigerweise durch ein Eprom oder Prom, wie z.B. die Typen 8755 oder ähnlichen oder durch diskrete Und-Odergatter und Zähler gebildet werden. Ist in der Logik 7 die Bedingung erfüllt, dass der eingestellte Sollwert niedriger als der eingestellte Ist-Druckwert ist, dann wird an den Steuerbaustein 6 nach Ablauf der Verzögerungszeit ein Signal weitergegeben und der Steuerbaustein 6 bildet, vorausgesetzt dass das Undgatter 21 es zulässt, den der ersten Frequenzstufe entsprechenden Code, die den Zählerbaustein 5, der im Mikroprozessor 8085, 8048 oder ähnlichem enthalten ist, auf die erste Stufe setzt und gleichzeitig die Anzeige 25 für die entsprechende Menge aktiviert. Der Zählerbaustein 5 schaltet die erste Frequenz des Frequenzgenerators 4 ein, der allerdings nur dann in seiner Frequenz schwingen kann, wenn die Lichtschranke 23, die zur Kontrolle der Luftblasen im Infusionsschlauch 15 dient, dies zulässt. In der Stufe 3 wird aus dem einphasigen Wechselstrom ein mehrphasiger Wechselstrom zum Betrieb des Schrittschaltmotors generiert. Ist nach Ablauf einer weiteren Zeitverzögerung in der Logik 7 immer noch der Sollwert niedriger als der gemessene Istwert, wird dieser Vorgang wiederholt und der Zählerbaustein 5 auf die nächste Stufe gesetzt, der Zählerbaustein 4, der ebenfalls im Mikroprozessor 8085, 8048, Z 8, 6000 oder ähnlichem enthalten ist, generiert dann die nächsthöhere Frequenz. Dies erfolgt solange bis zwischen Soll- und Istwert innerhalb der Hysteresegrenze Gleichgewicht herrscht. Die Pumpe 1, 2 läuft mit einer Geschwindigkeit, die der Menge entspricht, die der Patient zur Aufrechterhaltung eines bestimmten Blutdruckwertes braucht. Sinkt der Blutdruck unter den Sollwert, dann wird bei der ersten Hysteresegrenze nach der Zeitverzögerung der Zählerbaustein 5 und damit die Frequenzgenerierung 4 um eine Stufe zurückgesetzt. Sinkt der Wert während der Zeitverzögerung bis auf eine zweite prozentuelle Grenze von 20% wird im Steuerbaustein 6 der Codewert halbiert, worauf der Zählerbaustein 5 auf den halben Wert der zur Zeit gültigen Menge zurückspringt und der Frequenzgenerator 4 die halbe Frequenz und damit die Pumpe die halbe Drehzahl und die halbe Menge fördert. Sinkt der Druck weiter unter eine dritte Grenze, die weitere 20% unter dem Sollwert liegt, erhält der Steuerbaustein 6 von der Logik 7 ein Resetsignal, das er an den Zähler 5 weitergibt. Gleichzeitig wird die Alarmeinrichtung 22 aktiviert, der Zähler 5 geht auf Null zurück und der Oszillator 4 generiert keine Frequenz mehr. Die Pumpe bleibt infolgedessen sofort stehen. Befindet sich innerhalb der Lichtschranke im Schlauch 15 eine Luftblase, wird der Oszillator 4 ebenfalls gestoppt, wobei die generierte Menge jedoch nicht auf Null zurückgeht, sondern in derselben Grösse erhalten bleibt, nur der Oszillator wird ausgeschaltet und durch 24 Alarm gegeben, während bei Stop und Alarm durch das Undglied 21, also durch Fehlmengen und durch Fehldrücke und zu rasches Absinken des Istwertes die generierte Frequenz auf Null gesetzt wird und daher bei neuerlichem Anfahren mit dem Wert 1 begonnen wird. Hiedurch ist ein Maximum an Sicherheit gewährleistet und vor allen Dingen auch gewährleistet, dass keine Fehlmengen dem Patienten zugeführt werden. Denn wenn der Druck einen grossen Wert aufweist und die Durchflussmenge Null ist, bedeutet dies, dass die Kanüle verstopft ist. Die generierte Menge entspricht also nicht den Tatsachen. Umgekehrt, wenn die Durchflussmenge gross und der Druck sehr klein ist, bedeutet dies, dass die Kanüle aus der Vene herausgezogen wurde und die generierte Menge ebenfalls nicht den Tatsachen entspricht. Ist der Druck zu rasch abgesunken, bedeutet dies, dass der Patient für dieses Präparat nicht geeignet ist. Ist hingegen in der Leitung eine Luftblase, so stimmt die generierte Menge und kann daher in derselben Grösse beibehalten werden. Anstelle von Zählerbausteinen in den Bauteilen 3, 4, 5, 6 und 7 können auch Schieberegister oder einzelne Flip-Flop Verwendung finden, mit denen in bekannter Weise dieselben Funktionen erzielt werden. Die Bauteile 3, 4, 5, 6 und 7 können erfindungsgemäss aber auch durch einen Mikroprozessor wie 8085 in Verbindung mit einem Eprom 8755 und Rom 8155 oder durch einen Ein-chip-Mikroprozessor 8048 oder ähnlichem gebildet werden, da dieser im Prinzip dieselben Bauteile enthält und durch entsprechende Programmierung dieselben Funktionen erzielen kann.

Fig. 2 zeigt den Kurvenverlauf, wobei 32 die Mengenkurve ist, die stufenförmig langsam ansteigt. Durch entsprechende Codebildung im Steuerbaustein 6 kann dieser Anstieg linear oder auch exponential oder in einer anderen Kurvenform erfolgen. 33 zeigt die Blutdruckkurve des Patienten, die sich auf den entsprechenden Wert, der vorprogrammiert wurde, einstellt. 34 ist der vorgegebene Solldruckwert mit der Hysteresebegrenzung. Die Vorteile dieser Anordnung bestehen einmal darin, dass der Arzt und das Personal wesentlich entlastet werden, wobei insbesonders während Operationen und auf Intensivstationen, bei denen der Patient in bewusstlosem Zustand ist, durch die automatische Anpassung der Medikamentenmenge auf die entsprechende physikalische Grösse eine zusätzliche Sicherheit bieten. Besonders bei Schlaganfällen ist es schwierig, den Blutdruck unter Kontrolle zu bringen und gerade bei Blutungen im Gehirn ist es wichtig, dass der Blutdruck auf einem niedrigen Wert gehalten wird, um ein Weiterbluten zu verhindern. Aber auch die Beherrschung der Körpertemperatur ist von grosser Bedeutung besonders bei Erkrankungen, bei denen die vegetative Steuerung versagt. Durch die Mengen- und Drucksicherung sind Todesfälle, die durch Infusionszwischenfälle entstehen, weitgehend beseitigt.

**Patentansprüche**

1. Infusionspumpe zur intravenösen oder intraarteriellen Infusion von flüssigen Medikamenten mit elektrischem Antrieb (2) und elektronischer Mengensteuerung (3, 4, 5, 6, 7), wobei Mittel vorgesehen sind, die der Steuerung des Antriebs (2) der Infusionspumpe (1) dermassen zugeordnet sind, dass die geförderte Infusionsmenge in Abhängigkeit von den dem Patienten abgenommenen physikalischen Messgrössen verändert wird, dadurch gekennzeichnet, dass als elektronische Mengensteuerung (3, 4, 5, 6, 7) ein Mikroprozessor vorgesehen ist, der von einem einstellbaren Referenzwert als Sollwert und von einem vom Patienten abgegebenen Istwert des arteriellen Drucks beeinflusst wird und bewirkt, dass die Differenz aus Soll- und Istwert innerhalb einer prozentuell festgelegten Abweichung keine Änderung der digitalen Frequenzsteuerung des Antriebs (2) erzeugt, dass jedoch beim Überschreiten dieser Grenze nach oben oder unten eine Änderung der Frequenz um eine vorher festgelegte Stufe je nach der spontanen Wirkung des verwendeten Medikamentes nach oben oder nach unten nach Ablauf einer einstellbaren Zeitverzögerung erfolgt und dass bei Wirkung des Medikamentes über eine zweite vorher festgelegte prozentuelle Grenze innerhalb der Zeitverzögerung die Frequenz halbiert und bei Wirkung des Medikamentes über eine dritte vorher festgelegte prozentuelle Grenze die Frequenz auf Null gestellt wird und dass Einrichtungen vorgesehen sind, die bei Auftreten von Abweichungen, die ausserhalb der festgelegten Grenzen liegen, akustische und optische Alarmsignale abgeben und den Antrieb (2) abschalten.

2. Infusionspumpe nach Anspruch 1, dadurch gekennzeichnet, dass Mittel (16, 17, 18) vorgesehen sind, die die durchfliessende Infusionsmenge messen, in einen digitalen Wert umwandeln, dem Mikroprozessor (3, 4, 5, 6, 7) als Mengenwert zuführen, welcher diesen Mengenwert mit der eingestellten Frequenz vergleicht, wobei bei einer im Verhältnis zur Frequenz geringeren Infusionsmenge die Infusionspumpe (1) gestoppt und Alarm gegeben wird.

3. Infusionspumpe nach Anspruch 1 und 2, dadurch gekennzeichnet, dass der Pumpenantrieb (2) durch einen Schrittschaltmotor erfolgt.

4. Infusionspumpe nach Anspruch 1, dadurch gekennzeichnet, dass der Sollwert über eine Anzeigeeinrichtung einstellbar ist.

5. Infusionspumpe nach Anspruch 1, dadurch gekennzeichnet, dass als Infusionspumpe (1) eine Peristaltikschlauchpumpe Verwendung findet.

6. Infusionspumpe nach Anspruch 1, dadurch gekennzeichnet, dass der im Infusionsschlauch (15) herrschende Druck gemessen wird, mit einem oberen und unteren vorgegebenen Sollwert verglichen wird und bei Abweichung über die vorgegebenen Grenzen zum Stop und zum Alarm führt.

7. Infusionspumpe nach Anspruch 1, dadurch gekennzeichnet, dass eine Lichtschranke (23) zur Kontrolle von Lufteinschlüssen in der Infusionsflüssigkeit vorgesehen ist, die bei Eintreten einer Luftblase die Infusionspumpe (1) stoppt.

**Claims**

1. Infusion pump for intravenous or intra-arterial infusion of liquid medicaments, with electric drive (2) and electronic dosage control unit (3, 4, 5, 6, 7), containing devices which are coordinated to the drive (2) of the infusion pump (1) in such a way that the infusion volume delivered will be modified in dependence of the physical measuring data recorded from the patient, said infusion pump (1) being characterised by the fact that the electronic dosage control unit (3, 4, 5, 6, 7) is a microprocessor which is influenced by an adjustable reference value as control value and the actual value of arterial pressure recorded from the patient and which effects that while the variance of control value and actual value will not produce any modification of digital frequency control of the drive (2) within a fixed percentage of deviation, any positive or negative transgression of this limit will result in a modification of frequency by a previously fixed step in dependence on the spontaneous action of the medicament after expiration of an adjustable delay period, the frequency being halved if the medicament continues to be active beyond a second percentage limit previously fixed within this delay period and the frequency being reduced to zero if the medicament remains active even beyond a third percentage limit previously fixed, and containing further devices which emit acoustic and optical signals when deviations beyond the previously fixed limits occur and which will disconnect the drive (2).

2. Infusion pump according to claim 1, characterised by the presence of devices (16, 17, 18) which measure the infusion volume flowing through the pump, transforming the measured value into a digital value and feeding this quantitative value to the microprocessor (3, 4, 5, 6, 7), which compares this quantitative value with the previously set frequency and which will stop the infusion pump (1) and give alarm if the infusion volume is too small for the previously set frequency.

3. Infusion pump according to claims 1 and 2, characterised by the fact that the pump drive (2) consists of a step motor.

4. Infusion pump according to claim 1, characterised by the fact that the control value can be adjusted via a control unit.

5. Infusion pump according to claim 1, characterised by the fact that a peristaltic pump is used as infusion pump (1).

6. Infusion pump according to claim 1, characterised by the fact that the pressure in the infusion tube is measured and compared with present maximum and minimum control values, the pump being stopped and alarm being given in case of any deviation exceeding the presiously fixed limits.

7. Infusion pump according to claim 1, characterised by the presence of a light barrier (23) for the control of air locks in the infusion fluid, which will stop the infusion pump (1) as soon as an air bubble enters the pump.

## Revendications

1. Pompe à perfusion permettant de réaliser la perfusion intraveineuse ou intra-artérielle de médicaments liquides comportant un dispositif électrique d'entraînement (2) et un dispositif électronique de commande quantitative (3, 4, 5, 6 et 7) dans lequel il est prévu des moyens qui sont associés à la commande du dispositif d'entraînement (2) de la pompe à perfusion (1) de telle sorte que la quantité d'injection entraînée est modifiée en fonction des grandeurs de mesure physiques prélevées du patient, caractérisée par le fait qu'il est prévu comme dispositif électronique de commande quantitative (3, 4, 5, 6, 7) un microprocesseur qui est influencé par une valeur de référence réglable réglant une valeur de consigne et par une valeur réelle, tirée du patient, de la pression artérielle et agit de telle sorte que la différence entre la valeur de consigne et la valeur réelle se situant en deçà d'un écart fixé en pourcentage ne provoque aucune modification de la commande numérique de fréquence du dispositif d'entraînement (2), mais que dans le cas du dépassement de cette limite vers le haut ou vers le bas, il se produit une modification de la fréquence et ce d'un échelon fixé préalablement, en fonction de l'action spontanée du médicament utilisé, vers le haut ou vers le bas après l'écoulement d'un temps de retard réglable, et que lors de l'action du médicament audelà d'une seconde limite fixée préalablement en pourcentage, la fréquence est divisée de moitié pendant le temps de retard et que dans le cas de l'action du médicament au-delà d'une troisième limite fixée en pourcentage fixée préalablement, la fréquence est réglée à zéro, et qu'il est prévu des dispositifs qui, lors de l'apparition d'écarts situés à l'extérieur des limites fixées, délivrent des signaux d'alarme sonores et optiques et arrêtent le dispositif d'entraînement (2).

2. Pompe à perfusion suivant la revendication 1, caractérisée par le fait qu'il est prévu des moyens (16, 17, 18) qui mesurent la quantité d'injection administrée, la convertissent en une valeur numérique, l'envoient en tant que valeur quantitative au microprocesseur (3, 4, 5, 6, 7) qui compare cette valeur quantitative à la fréquence réglée, auquel cas la pompe à perfusion (1) est arrêtée et une alarme est délivrée dans le cas où la quantité d'injection est trop faible par rapport à la fréquence.

3. Pompe à perfusion suivant les revendications 1 et 2, caractérisée par le fait que le dispositif (2) d'entraînement de la pompe est un moteur pas-à-pas.

4. Pompe à perfusion suivant la revendication 1, caractérisée par le fait que la valeur de consigne est réglable par l'intermédiaire d'un dispositif indicateur.

5. Pompe à perfusion suivant la revendication 1, caractérisée par le fait qu'on utilise comme pompe à perfusion (1) une pompe tubulaire péristaltique.

6. Pompe à perfusion suivant la revendication 1, caractérisée par le fait que la pression régnant dans le tube à perfusion (15) est mesurée, et est comparée à une valeur de consigne prédéterminée supérieure ou inférieure et provoque l'arrêt et l'alarme dans le cas d'un écart par rapport aux limites prédéterminées.

7. Pompe à perfusion suivant la revendication 1, caractérisée par le fait qu'il est prévu un relais photo-électrique (23) servant à contrôler les inclusions d'air dans le liquide de la perfusion et qui arrête la pompe à perfusion (1) dans le cas de l'apparition d'une bulle d'air.

FIG 1

FIG 2